# EUROPEAN PATENT APPLICATION

(11) **EP 2 319 525 A1**
(43) Date of publication of application: **11.05.2011**
(21) Application number: 09175160.2
(22) Date of filing: 05.11.2009
(51) Int. Cl.: A61K 38/08

(54) **Composition for treating insulin resistance**

(71) Applicant: Universitätsklinikum Hamburg-Eppendorf, 20246 Hamburg (DE)
(72) Inventor: Benndorf, Ralf, D-20251, Hamburg (DE); Böger, Rainer, D-22459, Hamburg (DE)
(74) Representative: UEXKÜLL & STOLBERG

(57) **Abstract**

The present invention relates to the field of disorders and conditions which are characterized by an impaired insulin-induced glucose uptake. Specifically, the present invention provides a pharmaceutical composition comprising an agonist of the angiotensin receptor type-2 for the treatment or prevention of insulin resistance and/or disorders associated with insulin resistance. The present invention also relates to the use of an agonist of the angiotensin receptor type-2 for the preparation of a pharmaceutical composition for the treatment or prevention of insulin resistance and/or disorders associated with insulin resistance.

## Description

The present invention relates to the field of disorders and conditions which are characterized by an impaired insulin-induced glucose uptake. Specifically, the present invention provides a pharmaceutical composition comprising an agonist of the angiotensin receptor type-2 for the treatment or prevention of insulin resistance and/or disorders associated with insulin resistance. The present invention also relates to the use of an agonist of the angiotensin receptor type-2 for the preparation of a pharmaceutical composition for the treatment or prevention of insulin resistance and/or disorders associated with insulin resistance.

### BACKGROUND OF THE INVENTION

Obesity and its associated complications, such as insulin resistance, metabolic syndrome, and diabetes have become an increasingly burdensome health problem in the western world. For instance, the prevalence of obesity and the metabolic syndrome in the US population is steadily growing and has reached 25.7% and 22% in the year 2007, respectively. Consequently, the number of patients suffering from diabetes mellitus type 2 (also referred to as "non-insulin-dependent diabetes mellitus" or NIDDM) is expected to rise from 240 million in the year 2007 to 380 million in 2025. Moreover, the prevalence of the metabolic syndrome in adolescents is rising at an alarming rate and has reached 8.6% in the US population. Thus, innovative pharmacological strategies to prevent insulin resistance and new-onset diabetes are urgently needed to reduce the prevalence of diabetes and to prevent diabetes-associated complications, such as cardiovascular disease or diabetic nephropathy. Insulin resistance of skeletal muscle glucose transport represents a major defect in the normal maintenance of euglycemia and is often accompanied by a variety of metabolic and cardiovascular abnormalities, including hypertension, obesity, dyslipidemia, diabetes mellitus type 2, and atherosclerosis (Reaven GM, Diabetes 37:1595-1607, 1988; Reaven GM, Annu Rev Med 44: 121-131, 1993; Richey JM et al., Am J Physiol Endocrinol Metab 277: E920-E926, 1999; Zierath JR, et al., Diabetologia 43: 821-835, 2000). Hence, there is a need for new and innovative therapeutical concepts for improving glucose utilization in individuals afflicted with insulin resistance, in particular in order to prevent the onset of diabetes type 2 and other secondary disorders.

The renin angiotensin system (RAS) has been implicated in the pathogenesis of insulin resistance and diabetes (Henriksen EJ, Am J Physiol Regul Integr Comp Physiol, 293 (3) :R974-80, 2007). In this regard, the main effector of the RAS, the octapeptide angiotensin II, appears to play a major role. Angiotensin II signals primarily via two heptahelical receptors, the angiotensin receptor type-1 (also referred to as AT-1) and the angiotensin receptor type-2 (also referred to as AT-2). It is commonly accepted that angiotensin II promotes insulin resistance via activation of the AT-1 receptor (Henriksen EJ, see above; Shiuchi T, et al., Hypertension, 43(5):1003-10, 2004; Andreozzi F, et al., Circ Res., 94 (9) :1211-8, 2004). Consequently, inhibitors of the RAS, such as AT-1 receptor blockers, tend to improve insulin sensitivity and appear to reduce the prevalence of new-onset diabetes in large clinical trials involving diabetic and non-diabetic patients (Dahlöf B et al., Lancet, 359(9311):995-1003, 2002; Julius S, Lancet, 363 (9426):2022-31, 2004; Yusuf S et al., Circulation, 112 (1) :48-53, 2005). The role of the AT-2 receptor, however, is not well understood. A recent report proclaimed an inhibitory role of AT-2 receptor activation on insulin signaling (Cui TX, et al., M. Mol Endocrinol, 16(9):2113-23, 2002). In contrast, further reports were not able to detect an effect of AT-2 receptor antagonism (Hsieh PS, J Hypertens, 23(12):2209-17, 2005) or deficiency (Shiuchi T, et al., see above) on ARB-induced insulin sensitivity in rodent animal models.

It has now been surprisingly found by the present inventors that the stimulation of the AT-2 receptor enhances glucose uptake and the insulin response in skeletal muscle cells. Thus, agonists of the AT-2 receptor are suitable active agents which can be used for treating or ameliorating the severity of insulin resistance and disorders which involve or result from insulin resistance. Furthermore, it was found that a combination of AT-2 receptor agonism and AT-1 receptor antagonism synergistically enhances the glucose uptake effect and considerably surpassed the effect of AT-1 receptor antagonism alone on glucose uptake.

Accordingly, in a first aspect the present invention provides a pharmaceutical composition comprising an agonist of the angiotensin receptor type-2 for the treatment or prevention of insulin resistance and/or disorders associated with insulin resistance. In a further aspect, the invention relates to the use of an agonist of the angiotensin receptor type-2 for the preparation of a pharmaceutical composition for the treatment or prevention of insulin resistance and/or disorders associated with insulin resistance.

Angiotensin II is a potent peptide hormone and a primary regulator of the secretion of aldosterone from the adrenal cortex. It is also a potent vasoconstrictor which is involved in blood pressure regulation. Angiotensin II consists of 8 amino acids and has a molecular weight of 1046.9 Da. It is derived from the precursor molecule angiotensinogen, a 452 amino acid protein that is produced by the liver. In a first step, a decapeptide is cleaved from the angiotensinogen precursor by the proteolytical action of the angiotensinogenase renin. The decapeptide is referred to as angiotensin I, and the primary structure of angiotensin I is depicted in SEQ ID NO:1. In a subsequent step, angiotensin I is converted to angiotensin II by the removal of the two C-terminal amino acids from the decapeptide. This processing step is catalyzed by the angiotensin converting enzyme (ACE). The amino acid sequence of the resulting octapeptide, angiotensin II, is provided in SEQ ID NO:2. Angiotensin II acts through two types of G-protein coupled receptors which are referred to in the literature as angiotensin receptor type-1 (or AT-1 receptor) and angiotensin receptor type-2 (or AT-2 receptor), respectively. The human AT-1 and AT-2 receptor genes have been identified and extensively discussed in the scientific literature (Koike et al. (1994) Biochem Biophys Res Commun; 203(3):1842-50; Murphy et al. (1991) Nature; 351(6323):233-6; Bernstein and Berk. (1993) Am J Kidney Dis; 22(5):745-54; de Gasparo et al. (2000) Pharmacol Rev; 52(3):415-72).

The human AT-1 receptor gene is located on chromosome 3, in the region at about 149.90 to 149.94 Mb. The gene is composed of three exons, and at least five transcript variants have been described for this gene. Additional variants have been described, but their full-length nature has not been determined yet. The entire coding sequence is contained in the terminal exon and is present in all transcript variants. The AT-2 receptor gene consists of three exons and spans about 5 kb in the region Xq24-q25 on the X chromosome. Exons 1 and 2 of the gene encode for 5' untranslated mRNA sequence, whereas exon 3 comprises the entire uninterrupted open reading frame of the receptor protein.

The present invention provides evidence that the stimulation of the AT-2 receptor, for example by angiotensin II, enhances glucose uptake and insulin sensitivity in skeletal muscle cells. These effects have so far not been observed in response to angiotensin II or any other agonist of the AT-2 receptor. It has to be assumed that the AT-2 receptor stimulation by endogenously produced angiotensin II during AT-1 receptor antagonism may not be sufficient to uncover the AT-2 receptor-mediated effects on glucose uptake and insulin sensitivity as observed in the in vitro system described below. Indeed, during physiological conditions, plasma concentrations of angiotensin II in humans have been described to be in the picomolar range, whereas treatment with angiotensin receptor blockers roughly leads to a quadruplication of circulating angiotensin II levels as a result of interruption of the negative feedback loop at the renin-producing cells of the juxtaglomerular apparatus of the kidney. These concentrations are far below the Kd value (5 nM) which has been reported for the AT-2 receptor. Hence, AT-2 receptor-mediated effects on glucose utilization and insulin sensitivity may require a certain threshold stimulation which can be achieved only by application of a pharmacological AT-2 receptor agonist. Thus, this invention proclaims pharmacological AT-2 receptor stimulation as an innovative therapeutical intervention to facilitate peripheral glucose utilization and to enhance insulin sensitivity in patients with insulin resistance, when given alone or in combination with AT-1 receptor blockers.

The present invention also refers to the insight that antagonists of the AT-1 receptor promote glucose uptake in muscle cells, thereby counteracting insulin resistance. The use of AT-1 receptor blockers for improving insulin sensitivity and reducing the prevalence of new-onset diabetes has been previously contemplated (Dahlöf B et al., Lancet, 359(9311):995-1003, 2002; Julius S, Lancet, 363 (9426):2022-31, 2004; Yusuf S et al., Circulation, 112 (1) :48-53, 2005). According to a particular embodiment of the present invention, it is preferred to block the AT-1 receptor while, at the same time, stimulating the AT-2 receptor to maximize the therapeutic outcome of AT-2 agonism.

The invention provides pharmaceutical compositions which comprise, as an active agent, an agonist of the AT-2 receptor. As used herein, an agonist of the AT-2 receptor refers to a molecule which binds and activates signal transduction via the AT-2 receptor to the cell. As used herein the term agonist shall also include the natural ligand of the receptor, angiotensin II, or derivatives thereof. AT-2 receptor agonists of the invention other than angiotensin II may bind to the AT-2 receptor with a higher or lower affinity compared to angiotensin II.

According to a preferred embodiment, the AT-2 receptor agonist is the below CGP-42112A (IUPAC name: 3S)-2-[[(2S)-1-[(2S)-2-[[(2S)-6-[[(2S)-5-(diaminomethylideneamino)-2-(phenylmethoxy-carbonylamino)pentanoyl]amino]-2-[[(2S)-3-(4-hydroxyphenyl)-2-(pyridine-3-carbonylamino)propanoyl]amino]hexanoyl] amino] -3-(3H-imidazol-4-yl)propanoyl]pyrrolidine-2-carbonyl]amino]-3-methylpentanoic acid). The structural formula of CGP-42112A is depicted below:

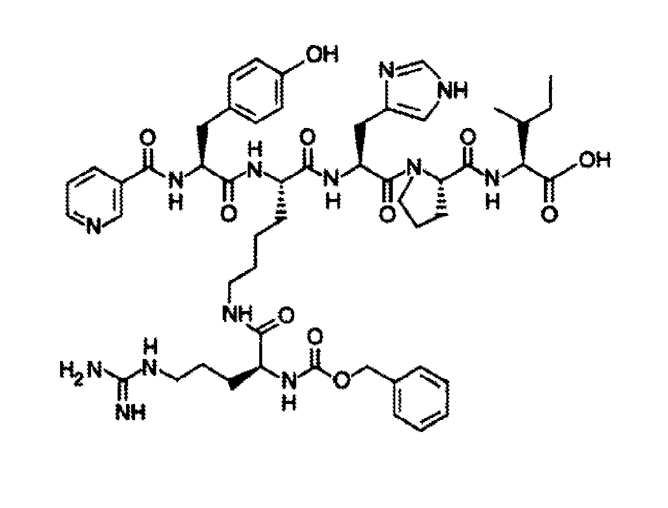

The use of CGP-42112A as an AT-2 receptor agonist is described in Macari et al., Eur J Pharmacol. 1993; 249:85-93 and Macari et al., Eur J Pharmacol. 1994; 259:27-36. The K_{I} values of CGP-42112A as determined in the spleen and in the fetus of rats have been reported in the publication of Ciuffo and Saavedra (1995), J Pharmacol Exp Ther, 274(3):1129-34.

It will be appreciated by those skilled in the art that the structure of CGP-42112A may be substituted or otherwise modified in one or more positions, as long as this substitution or modification does not significantly affect the agonistic effect of the compound and does not result in a derivative that is pharmacologically unacceptable due to toxicity issues. For example, one or more hydrogens of the C-H bonds of the heterocyclic ring system may be replaced by halogen, e.g. chlorine, bromine or iodine atoms. Further, the hydrogens of the C-H bonds may also be replaced by alkyl groups, e.g. methyl, ethyl or propyl groups.

Other suitable AT-2 receptor agonist for use in the present invention have been described in the prior art and include those disclosed in US 2004/0167176.

As outlined above, the pharmaceutical compositions contemplated herein for the treatment of insulin resistance preferably also comprise an antagonist of the angiotensin receptor type-1. As used herein, an antagonist of the AT-1 receptor for use in the present invention is a molecule which binds to the AT-1 receptor without inducing a biological response upon binding to a receptor. Binding of an antagonist to the receptor inhibits the biological response that is mediated by the receptor agonist, such as the natural ligand.

Several different antagonists of the AT-1 receptor are known in the art, and some of them are also approved for being used as therapeutic agents in the treatment of several diseases or disorders in humans. Antagonist of the angiotensin receptor type-1 which are approved for use in humans and which may be used in the present invention, include but are not limited to losartan (2-butyl-4-chloro-1-{[2'-(1H-tetrazol-5-yl)biphenyl-4-yl]methyl}-1H-imidazol-5-yl)methanol), candesartan (2-ethoxy-1-({4-[2-(2H-1,2,3,4-tetrazol-5-yl)phenyl]phenyl}methyl)-1H-1,3-benzodiazole-6-carboxylic acid), eprosartan (4-({2-butyl-5-[2-carboxy-2-(thiophen-2-ylmethyl)eth-1-en-1-yl]-1H-imidazol-1-yl}methyl)benzoic acid), irbesartan (2-butyl-3-({4-[2-(2H-1,2,3,4-tetrazol-5-yl)phenyl]phenyl}methyl)-1,3-diazaspiro[4.4]non-1-en-4-one), olmesartan ((5-methyl-2-oxo-2H-1,3-dioxol-4-yl)methyl 4-(2-hydroxypropan-2-yl)-2-propyl-1-({4-[2-(2H-1,2,3,4-tetrazol-5-yl)phenyl]phenyl}methyl)-1H-imidazole-5-carboxylate), tasosartan (2,4-dimethyl-8-{[2'-(1H-tetrazol-5-yl)biphenyl-4-yl]methyl}-5,8-dihydropyrido[2,3-d]pyrimidin-7(6H)-one), telmisartan (2-(4-{[4-methyl-6-(1-methyl-1H-1,3-benzodiazol-2-yl)-2-propyl-1H-1,3-benzodiazol-1-yl]methyl}-phenyl) benzoic acid), and valsartan ((S)-3-methyl-2-[N-({4-[2-(2H-1,2,3,4-tetrazol-5-yl)phenyl]phenyl}methyl)pentanamido]butanoic acid). Again, it will be appreciated that the structure of the above-listed antagonists of the angiotensin receptor type-1 may be substituted or otherwise modified in one or more positions, as long as this substitution or modification does not significantly affect the antagonistic effect of the compound and does not result in a derivative that is pharmacologically unacceptable due to toxicity issues. For example, one or more hydrogens of the C-H bonds of the heterocyclic ring systems of the compounds may be replaced by halogen, e.g. chlorine, bromine or iodine atoms. Further, the hydrogens of the C-H bonds of the compounds may also be replaced by alkyl groups, e.g. methyl, ethyl or propyl groups.

The AT-1 receptor antagonists of the invention can be competitive or non-competitive antagonists. Competitive antagonists of a given receptor bind to said receptor at the same binding site as the natural ligand, i.e. angiotensin II. Thus, a competitive antagonist of the AT-1 receptor competes with angiotensin II or other agonists of the AT-1 receptor for the same binding site on the receptor. Upon binding of the antagonist to the AT-1 receptor, binding of angiotensin II or other agonists is blocked. For example, eprosartan is a competitive antagonist which competes with angiotensin II for binding to the AT-1 receptor. A competitive antagonist can be a reversibly binding or an irreversibly binding antagonist. The effects of a reversibly binding competitive antagonist may be reduced by increasing the concentration of angiotensin II or any other agonists of the AT-1 receptor.

Alternatively, the receptor can be a non-competitive antagonist. Non-competitive antagonists do not compete with angiotensin II or other AT-1 receptor agonists for the same binding site, but instead bind to a distinct binding site of the receptor molecule. These antagonists are often called allosteric antagonists. A non-competitive antagonist, which has bound to the AT-1 receptor, may result in a decreased affinity of said receptor for angiotensin II or other agonists. Alternatively binding of the non-competitive AT-1 receptor antagonist may prevent conformational changes in the receptor required for receptor activation after binding of an agonist. A non-competitive antagonist can be reversibly binding or irreversibly binding. For example, valsartan and irbesartan are non-competitive antagonists which irreversibly bind to the AT-1 receptor.

It is further preferred that the agonist of the AT-2 receptor is selective for the AT-2 receptor, which means that it exhibits at least about a 2-fold, more preferably at least about 4-fold, 6-fold, 8-fold, 10-fold, 20-fold, 50-fold, or even about 100-fold greater binding affinity for the AT-2 receptor than for the AT-1 receptor or any other receptor which is unrelated to the renin angiotensin system. Conversely, it is also preferred that the antagonist of the AT-1 receptor is one that is selective for this receptor, which means that it exhibits at least about a 2-fold, more preferably at least about 4-fold, 6-fold, 8-fold, 10-fold, 20-fold, 50-fold, or even about 100-fold greater binding affinity for the AT-1 receptor than for the AT-2 receptor. Binding affinities can be determined, for example, by measuring the dissociation constant (Kd value). According to the invention, it is particularly preferred that the agonist of the AT-2 receptor does not bind or activate the AT-1 receptor to a significant extent.

The above discussed agonists of the AT-2 receptor and the antagonists of the AT-1 receptor can be, for example, antibodies or functional fragments thereof, small molecule organic compounds or polypeptides, peptides or peptidomimetics. Preferably, the agonists or antagonists of the invention are small organic molecules or peptides/polypeptides.

According to a particularly preferred aspect of the invention, the agonist of the angiotensin receptor type-2 and/or the antagonist of the AT-1 receptor are peptides (either linear or cyclic) or polypeptides molecule. For example, an agonistic or antagonistic peptide may be derived by modifying the angiotensin II octapeptide. It has been shown that the minimum active fragment of angiotensin II is the C-terminal pentapeptide (Moore et al. (1993), Int. J. Peptide Protein Res., 42:445-449). Thus, an agonist of the angiotensin receptor type-2 and/or an antagonist of the AT-1 receptor can be modified or unmodified fragments derived from the angiotensin II peptide, and in particular modified or unmodified fragments which comprise the C-terminal pentapeptide of angiotensin II. Modifications of the angiotensin II octapeptide can include one or more amino acid additions, deletions or substitutions. Deletions can be made at any position of the octapeptide, as long as at least a part of the agonistic activity is retained by the modified peptide. If amino acid substitutions are made, the substitutions are preferably conservative amino acid substitutions. A conservative amino acid substitution is a substitution in which an amino acid residue is replaced with another residue having a chemically similar side chain. Families of amino acid residues having similar side chains have been defined in the art. These families include amino acids with acidic side chains (e.g., aspartic acid, glutamic acid), basic side chains (e.g., lysine, arginine, histidine), uncharged polar side chains (e.g., glycine, asparagine, glutamine, serine, threonine, tyrosine, cysteine), non-polar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

Conservative amino acid substitutions also include the replacement of a non-derivatized amino acid by a chemically derivatized residue (i.e. an amino acid which has been modified by reaction of a functional side group), provided that the resulting peptide or polypeptide still exhibits the desired agonistic or antagonistic activity. Derivatized amino acid include for example, those amino acids in which one or more free amino groups have been modified to form formyl groups, chloroacetyl groups, or carbobenzoxy groups. Similarly, amino acids are included, in which one or more free carboxyl groups have been modified to form salts, methyl or ethyl esters. Free hydroxyl groups may be derivatized to form O-acyl or O-alkyl derivatives.

The agonistic or antagonistic peptide or polypeptide molecules may be prepared by any suitable method known in the art for producing peptides or polypeptides. Both ribosomal and non-ribosomal strategies are available in the art. Polypeptides containing more than about 50 amino acid residues are often prepared via recombinant expression systems using a host organism, such as bacteria or yeast. A typical approach comprises culturing a host cell, such for example bacterial cells or yeast cells, containing a suitable expression vector comprising a nucleic acid sequence encoding the desired agonistic peptide, under conditions suitable for the expression of said peptide or polypeptide, and recovering said peptide or polypeptide from the cell culture.

Alternatively, the agonistic or antagonistic peptides or polypeptides may be prepared as fusion peptides or fusion polypeptides. As used herein, a fusion peptide or fusion polypeptide refers to a fusion of a first amino acid sequence comprising the agonistically active peptide of interest which is N-terminally or C-terminally linked to a second amino acid sequence. The second amino acid sequence may be, for example, an affinity tag, i.e. an amino acid sequence that is fused to the 5' or 3' end of the agonistic peptide or polypeptide and which exhibits an increased affinity to another compound, thereby allowing purification of the fusion protein. Preferably, the tag sequence is removed from the agonist peptide of interest after purification, for example by providing a proteolytic cleavage site between the agonist/antagonist and the affinity tag.

Smaller peptides are usually prepared by chemical synthesis. For example, the peptides may be chemically synthesized by solid phase or liquid phase methods. Protocols for solution-phase chemical synthesis of peptides have been described (see, for example, Andersson et al., Biopolymers 55:227-250, 2000). For solid phase synthesis the technique described by Merrifield (J. Am. Chem. Soc., 1964, 85, 2149-2154) may be used. In that approach, the growing peptide is anchored on an insoluble resin, and unreacted soluble reagents are removed by filtration or washing steps without manipulative losses. Solid phase peptide synthesis can be readily performed by use of automated devices.

The agonists of the AT-2 receptor and/or the antagonists of the AT-1 receptor can also be antibodies or functional fragments thereof. Antibodies which specifically bind to angiotensin receptors AT-1 and AT-2 are known in the art and are available, for example, from Santa Cruz Biotechnology Inc., Santa Cruz, California, USA, and also from other suppliers. It can be readily tested, for example, by use of the methods described below, whether these antibodies display agonistic or antagonistic properties for any of the receptors of the renin angiotensin system. For example, stimulation of angiotensin II type 1 receptor by an agonistic antibody was reported in the literatur (Dragun D, Nephrol Dial Transplant (2007), 7: 1819-22). Antibodies which are agonists to AT-2 receptor or antagonists to the AT-1 receptor can be identified as described herein and used in the methods and compositions of the present invention.

As used herein, the term "antibody" includes monoclonal antibodies, polyclonal antibodies, multispecific antibodies (for example, bispecific antibodies), anti-idiotypic antibodies, chimeric antibodies, and humanized antibodies, as long as they exhibit the desired agonistic or antagonistic activity. In particular, the term is to be understood as comprising any kind of artificial antibody molecule that was prepared by grafting techniques and similar methods. The antibodies for use in the present method may be of any isotype class, such as IgG, IgM, IgA, IgE, and IgD as well as their subclasses, e.g., IgG1, IgG2 and the like.

Apart from whole antibodies, the present invention also refers to antigen-binding fragments of an antibody which specifically bind to the AT-1 or AT-2 receptor. As used herein, an antigen-binding fragment of an antibody according to the invention is a fragment which retains at least a part of the agonistic or antagonistic activity of the complete antibody. Antigen-binding fragments of the invention may comprise Fv, Fab, F(ab') and F(ab')2 fragments. Further included by the term "antigen-binding fragment" are single chain Fv antibody fragments (which are also designated "scFv antibodies herein) and disulfide-linked Fv fragments (dsFv). Methods for the recombinant production of antibody fragments have been described in the prior art and comprise, for example, techniques such as phage display or ribosome display. The recombinant antibody fragments produced by these methods may be purified and tested for their binding affinity to the AT-1 or AT-2 receptors, and also for their agonistic or antagonistic effects with respect to these receptors.

In a further preferred embodiment, the agonists of the AT-2 receptor and/or the antagonists of the AT-1 receptors are small molecules, i.e. small non-polymeric organic compounds having a molecular weight below about 5000 Da, preferably below about 2000 Da, more preferably below about 1500 Da, and most preferably below about 1000 Da.

Potential agonistic or antagonistic effects of candidate compounds, such as peptide/polypeptide molecules, antibodies/antibody fragments, or small molecules, on the AT-1 or AT-2 receptor, respectively, can be identified by commonly used methods. For example, peptide, antibody, or small molecule libraries can be screened for compounds which exhibit binding to at least one of the receptors. In a subsequent step, these compounds are further analyzed for receptor-mediated biological activity.

For example, the identification of an AT-2 agonist can be achieved by screening in a first step a library, e.g. a small molecule, antibody or peptide library, for compounds having the ability to bind to the AT-2 receptor using standard radio-ligand binding protocols for the AT-2 receptor (see, for example, Wolf et al., Hypertension (1996); 27(4):897-905 or Wu et al., J Med Chem (2006); 49(24):7160-8). Compounds, which display high affinity binding to the AT-2 receptor, are tested in established assays for their ability to induce AT-2 receptor-mediated biological activity. AT-2 receptor stimulation has been shown to induce neurite outgrowth in neural cell lines, NG108-15 cells (ATCC #HB12317) and PC12W cells (ATCC #CRL-1721) (Laflamme et al., J Biol Chem (1996; 271:22729-22735; Meffert et al., Mol. Cell. Endocrinol., (1996); 122:59-67). Hence, AT-2 receptor-binding compounds can be tested for their ability to induce neurite outgrowth of neural cell lines via AT-2 receptor activation. Moreover, specific pharmacological inhibitors of the AT-2 receptor, such as PD123319, should be able to block the activity and the biological effects of these molecules on neurite outgrowth.

In a second step, candidate AT-2 agonists which have shown to stimulate AT-2 receptor-mediated neurite outgrowth could be tested for AT-2 receptor agonism using the myoblast cell line L6 (ATCC #CRL-1458). As described in detail below, AT-2 receptor agonism induces glucose uptake in differentiated L6 myotubes in an insulin-independent fashion. Hence, this assay type also represents a suitable system for identification of, for example, small organic molecules with the potential to stimulate the AT-2 receptor. Moreover, the AT-2 receptor-mediated effect on glucose uptake in L6 myotubes is considerably enhanced by AT-1 receptor blockade and abolished by the specific AT-2 receptor antagonist PD123319. Hence, AT-2 receptor-driven insulin-independent glucose uptake in the myoblast cell line L6 represents a second suitable read-out for the identification of drug candidates with the ability to stimulate the AT-2 receptor. In conclusion, AT-2 receptor-activating molecules can be identified efficiently by the combined characteristics of high affinity binding to the AT-2 receptor and the ability to induce well-characterized AT-2 receptor-mediated effects in vitro.

The present invention provides pharmaceutical compositions useful for treating or preventing insulin resistance as well as diseases or disorders associated with insulin resistance. The preparation of pharmaceutical compositions containing an agonist of the angiotensin receptor type-2 and, optionally, an antagonist of the angiotensin receptor type-1, as an active ingredient is well within the ordinary skills of those working in the field of pharmaceutics. Such pharmaceutical compositions can be prepared for injection, e.g. in the form of liquid solutions or suspensions. Alternatively, the active ingredient may be emulsified in the compositions. The active ingredient can be mixed with excipients which are pharmaceutically acceptable and compatible with the active ingredient and in amounts suitable for use in the human patients.

In particular, the pharmaceutical compositions of the present invention may contain a physiologically acceptable carrier together with the agonistic and/or antagonistic molecules dissolved or dispersed therein. As used herein, the term "pharmaceutically acceptable carrier" comprises, but is not limited to, water, saline, Ringer's Solutions, dextrose solution, and 5% human serum albumin. Liposome-based carriers and nonaqueous vehicles such as fixed oils may also be used. Further examples of suitable carriers for compositions comprising the agonistic and/or antagonistic compounds of the invention are described in standard textbooks, for example, in "Remington's Pharmaceutical Sciences", Mack Pub. Co., New Jersey (1991). In addition to the carrier, the composition may also contain further compounds, such as wetting agents, emulsifying agents, pH buffering agents, stabilizers, dyes and the like, as long as these compounds do not interfere with the desired biological activity of the agonistic and/or antagonistic compounds. According to a preferred embodiment of the invention, the pharmaceutical compositions of the present invention contain peptidic vectors for internalizing an agonistic and/or antagonistic peptide into the cells or tissues at the site where the therapeutic effect is desired. Suitable peptidic vectors are described, for example, in US-Patent No. 6,080,724.

The pharmaceutical composition provided by the present invention will be formulated to be compatible with the intended route of administration. Different routes of administration are feasible for providing the AT-2 receptor agonists to the patient in need of treatment. Accordingly, the pharmaceutical composition of the present invention can be formulated for oral, rectal, nasal or parenteral administration (including subcutaneous, intramuscular, intravenous and intradermal administration).

The pharmaceutical composition can be formulated for parenteral administration, for example, for intravenous, intrathe-cal, intradermal, subcutaneous, topical, transmucosal, or rectal administration. Solutions or suspensions used for parenteral, intradermal or subcutaneous application usually comprise a sterile diluent such as water, saline solution, fixed oils, polyethylene glycols, glycerine, propylene glycol or other synthetic solvents. The solutions or suspensions may further comprise antibacterial agents such as benzyl alcohol or methyl parabens, antioxidants such as ascorbic acid or sodium bisulfite, chelating agents such as EDTA, buffers such as acetates, citrates or phosphates, and agents for the adjustment of tonicity such as sodium chloride or dextrose. The pH can be adjusted with suitable acids or bases, such as hydrochloric acid or sodium hydroxide. The pharmaceutical compositions can be enclosed in ampoules, disposable syringes or multiple dose vials made of glass or plastic.

Pharmaceutical compositions suitable for injection normally include sterile aqueous solutions or dispersions and sterile powders for the extemporaneous preparation of sterile injectable solutions or dispersions. The composition intended for injection must be sterile and should be fluid in order to allow a convenient handling in a syringe. The composition should be stable under the conditions of manufacturing and storage and is preferably preserved against the contaminating action of microorganisms such as bacteria and fungi, for example, by including parabens, chlorobutanol, phenol, ascorbic acid, thimerosal, and the like into the composition. For intravenous administration, suitable carriers may comprise physiological saline, bacteriostatic water, Cremophor EL™ (BASF) or phosphate buffered saline (PBS). The carrier may also be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Sterile injectable solutions can be prepared by incorporating the receptor agonist and/or antagonist in the required amount in an appropriate solvent with one or more of the above mentioned ingredients followed by sterile filtration. Generally, dispersions are prepared by incorporating the active compound into a sterile vehicle that contains a basic dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile-filtered solution thereof.

Administration of the AT-2 receptor agonist and/or the AT-1 receptor antagonist may also be achieved by transmucosal or transdermal delivery (Chen et al., Nature Biotechnology 24, 455-460 (2006)). For transmucosal or transdermal administration, the pharmaceutical composition of the invention, which comprises the agonist/antagonist, will also contain penetrants appropriate for the barrier to be permeated. Such penetrants are known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art. Preferably, the compounds are prepared in the form of suppositories, with conventional suppository bases such as cocoa butter and other glycerides for rectal delivery.

In one embodiment, the active compounds are prepared with carriers that will protect the active compounds against elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparing a controlled release formulation are well-known in the art. Furthermore, sustained-release compositions can be prepared. Suitable examples of sustained-release preparations include semi-permeable matrices of solid hydrophobic polymers, which matrices are in the form of shaped articles, e.g., films or microcapsules. Examples of sustained-release matrices include polyesters, hydrogels, polylactides, copolymers of L-glutamic acid and ethyl-L-glutamate, non-degradable ethylenevinyl acetate, degradable lactic acid-glycolic acid copolymers and the like.

The pharmaceutical composition according can be formulated for administration by injection or continuous infusion, for example, by continuous infusion for a period of between 2 and 21 days, more preferably a period of between 10 and 14 days.

The pharmaceutical composition of the invention, in particular compositions comprising small organic molecules as an active agent, will preferably be formulated for oral administration, for example, in the form of granules, powders, tablets, pills, capsules, gelcaps, emulsions, suspensions or solutions. The preparation of solid dosage forms can be achieved by mixing the active agent or a salt thereof with an appropriate edible carrier, such as microcrystalline cellulose, methylcellulose, hydroxypropyl methylcellulose, casein, albumin, mannit, dextran, saccharose, lactose, sorbitol, agar, alginate, pectine, collagen, starch or gelatine. The solid dosage form may also include one or more excipients, such as surfactants, binders, thickeners, diluents, lubricants, disintegrants, and glidants. Further, the solid dosage form can comprise antioxidants (for example, ascorbic acid, tocopherol or cysteine), preservatives (for example, parabene), coloring or flavoring agents, and the like. Mixing of the above components may involve dry granulation, wet granulation, or direct compression.

The pharmaceutical composition of the invention can also be formulated as a fluid dosage form. Fluid dosage forms which are suitable for oral administration can be formulated, for example, as emulsions, treacle, suspensions or solutions. These formulations can be prepared by using a sterile fluid as a carrier (for example oil, water, alcohol or combinations thereof). Suitable oils for use in fluid compositions include, for example, olive oil, sesame oil, peanut oil, rapeseed oil, and maize oil. Suitable alcohols include, ethanol, isopropyl alcohol, hexadecyl alcohol, glycerin and propylene glycol. Pharmaceutically acceptable surfactants, suspending agents, or emulsifiers can be added. Suitable suspending agents for use in the present invention are methyl cellulose, sodium carboxymethyl cellulose, hydroxypropyl methyl cellulose, polyvinylpyrrolidone, alginates, chitosan, polyoxyethylene- and polyoxypropylene ethers. Suitable surfactants include anionic, cationic or non-ionic surfactants. Suitable anionic surfactants include sodium lauryl sulfate, sodium laurate, sodium stearate, potassium stearate, sodium oleate, and the like. Suitable cationic surfactants include benzalkonium chloride, bis-2-hydroxyethyl oleyl amine, or the like. Suitable non-ionic surfactants include polyoxyethylene sorbitan fatty acid esters, fatty alcohols such as lauryl, cetyl and stearyl alcohols, glyceryl esters and the like. Suitable emulsifiers include, foe example, ethyl carbonate, ethyl acetate, benzyl benzoate, propylene glycol, 1,3-butylene glycol, dimethyl formamide.

The compositions contemplated by the invention can also include pharmaceutically acceptable salts of the agonistic or antagonistic compounds of the invention. As used herein, pharmaceutically acceptable salts include acid addition salts (formed e.g. with the free amino groups of a peptide or polypeptide) that are formed with inorganic acids such as, for example, hydrochloric or phosphoric acids, or with organic acids, such as acetic acid or tartaric acid and the like. Salts formed with the free carboxyl groups can also be derived from inorganic bases such as, for example, sodium, potassium, ammonium, calcium or ferric hydroxides, and such organic bases as isopropylamine, trimethylamine, 2-ethylamino ethanol, histidine, procaine and the like.

The pharmaceutical compositions of the invention comprise a therapeutically effective amount of an agonist of the AT-2 receptor, optionally in combination with a therapeutically effective amount of an antagonist of the AT-1 receptor. As used herein, the term "therapeutically effective amount" means that the AT-2 receptor agonist has to be present in the composition in an amount that is sufficient for reducing insulin resistance and/or enhancing glucose uptake in a patient suffering from insulin resistance. Similarly, the antagonist of the AT-1 receptor has to be present in an amount that is sufficient for reducing insulin resistance and/or enhancing glucose uptake in said patient having insulin resistance. Due to the synergistic effect upon use of a combination of an AT-1 receptor antagonist and an AT-2 receptor agonist, the term also refers to embodiments in which an amount of a certain agonist and/or antagonist would not lead to an observable biological reaction in the patient upon use of the compound alone, but which results in a detectable therapeutical effect when used in combination.

The dosage ranges for the agonists and/or antagonists for administration into a patient, preferably a human, will largely depend on the compound class of the agonists and/or antagonists, as well as their formulation. The skilled person will routinely find amounts of the active ingredients that are high enough to produce the desired therapeutic effect, i.e. reducing insulin resistance. The dosage will not be so high as to cause adverse side effects that are harmful for the patient to be treated. It will be appreciated that the concrete amount of the agonistic and/or antagonistic compounds to be administered to the patient will depend on several other factors, such as age and weight of the patient, as well as the nature and severity of the medical symptoms to be treated. The amount will in any individual case be determined by one of ordinary skill in the art using routine experimentation.

For example, a therapeutically effective amount of an agonistic and/or antagonistic peptide molecule or a small molecule is typically an amount such that, when administered in a physiologically tolerable composition, is sufficient to achieve a plasma concentration in the patient from about 0.2 µg/ml to about 200 µg/ml, preferably from about 1 µg/ml to about 100 µg/ml, wherein 50 µg/ml is particularly preferred. Hence, the preferred plasma concentration of a peptide or small molecule agonist of the AT-2 receptor or a peptide or small molecule antagonist of the AT-1 receptor after administration will be in a range from about 0.005 mM to about 5 mM, and preferably about 0.5 mM to 2 mM, wherein a concentration of 1 mM is particularly preferred. Therefore, according to a preferred embodiment, the dosage per body weight can vary from about 0.15 mg per kg body weight of the patient to about 400 mg per kg body weight of the patient, and preferably from about 0.5 mg per kg body weight of the patient to about 200 mg per kg body weight of the patient, used in one or more dose administrations daily. Thus, according to a preferred embodiment of the invention, the therapeutically effective amount of the agonistic and/or antagonistic peptide or small molecule ranges from 0.15 mg per kg body weight of the patient to about 400 mg per kg body weight of the patient.

Where the AT-2 receptor agonist and/or the AT-1 receptor antagonist is an antibody, such as a monoclonal antibody, a therapeutically effective amount is typically an amount such that when administered is sufficient to achieve a plasma concentration of from about 0.05 µg/ml to about 500 µg/ml, preferably from about 5 µg/ml to about 100 µg/ml, more preferably from about 10 µg/ml to 50 µg/ml, for example, about 20 µg/ml, about 30 µg/ml, or about 40 µg/ml. Accordingly, the dosage can vary from about 0.05 mg/kg to about 500 mg/kg, preferably from about 0.5 mg/kg to about 100 mg/kg, more preferably from about 1.0 mg/kg to about 50 mg/kg, for example, about 5 mg/kg, about 10 mg/kg, about 20 mg/kg, about 30 mg/kg, about 40 mg/kg, about 50 mg/kg, about 60 mg/kg, about 70 mg/kg, about 80 mg/kg, or about 90 mg/kg. The dosage amount can be administered in one or more dose administrations daily, either for one or for several days, e.g. for 2, 3, 4, 5, 6, 7 or more days. Where the antagonist is in the form of a fragment of an antibody, preferably from a monoclonal antibody, the amount to be administered will be somewhat lower than the above recited amounts; the skilled person will readily determine suitable dosage amounts for administration of such fragments.

According to the invention, it was found that the AT-2 receptor agonist, when used in a combination therapy together with an AT-1 receptor antagonist, leads to an enhanced therapeutic response which is associated with an improved treatment outcome for insulin resistance, thereby preventing disorders associated with insulin resistance, in particular diabetes mellitus type 2. As used herein, the term "combination therapy" shall mean treatment of a subject in need thereof by administering one or more AT-2 receptor agonists in combination with one or more AT-1 receptor antagonists. The agonistic and antagonistic compounds can be administered simultaneously, sequentially, separately or in a single pharmaceutical formulation. Where the one or more AT-2 receptor agonists and the one or more AT-1 receptor antagonists are administered in separate dosage forms, the number of dosages administered per day for each compound may be the same or different. Further, the AT-2 receptor agonist(s) and the AT-1 receptor antagonist(s) may be administered via the same or different routes of administration. Also, the AT-2 receptor agonist(s) and the AT-1 receptor antagonist(s) may be administered according to simultaneous or alternating regimens, at the same or different times during the course of the therapy, concurrently in divided or single forms.

The pharmaceutical compositions disclosed herein are suitable for being used in the treatment or prevention of insulin resistance and/or disorders associated therewith, as they improve glucose utilization and insulin sensitivity of cells, such as muscle cells. They enhance glucose uptake by these cells, thereby reducing the blood glucose level. By reducing the blood glucose level, the compositions are suitable to effectively treat and/or prevent disorders which result from the increased blood glucose level. Such disorders include, in particular, diabetes mellitus type 2 and type 1, and disorders following from diabetes mellitus, such as diabetic retinopathy, diabetic nephropathy, blindness, cardiovascular abnormalities, hypertension, obesity, dyslipidemia, atherosclerosis, renal failure, neuropathy, syndrome X, acromegaly, and Cushing's syndrome.

Thus, the use of an agonist of the AT-2 receptor represents an innovative strategy for the treatment of insulin resistance in patients which suffer from one or more of the above diseases or disorders, or are at a pre-disease state, such as a prediabetic state. Preferably, the human patient who receives the AT-2 receptor agonist (optionally in combination with an AT-1 receptor antagonist for treating and/or preventing the above diseases or disorders is a child or adolescent, i.e. 5-18 years old, more preferably 8 or 10-18 years old. The AT-2 receptor is strongly expressed in during infancy and adolescence, whereas expression of the receptor is markedly reduced in adults. Thus, treatment of childs and adolescents should be particularly efficient in these age groups.

### BRIEF DISCRIPTION OF THE DRAWINGS

Figure 1 depicts the results obtained from AT-2 receptor stimulation experiments using certain AT-1/AT-2 receptors and combinations thereof.
Figure 2 shows the results obtained from AT-2 receptor stimulation experiments using further AT-1/AT-2 receptors and combinations thereof.
Figure 3 depicts the results from long-term stimulation experiments of the AT-2 receptor.

### EXAMPLES

### Example 1: Culturing of L6 myoblasts

The data presented below were generated in the rodent myoblast cell line L6 (ATCC #CRL-1458). L6 myoblasts are able to differentiate into myotubes which possess classical characteristics of skeletal muscle and represent a common *in vitro* system to detect the impact of pharmacological interventions on insulin sensitivity (Ueyama et al., Am J Physiol Endocrinol Metab, 277(3 Pt 1):572-8, 1999). L6 myotubes express both the AT-1 and the AT-2 receptor at relevant levels making these cells an ideal model to test the interference of both receptors with basal and insulin-mediated glucose uptake. L6 myoblasts were maintained as proliferating myoblasts in DMEM high glucose medium supplemented with 10% FCS, 4 mM L-glutamine, 1 mM pyruvate at 37°C in the presence of 5% CO₂. The seeding density used for the experiments was 2 x 10⁴ cells/cm² on 1% gelatincoated dishes. To induce differentiation of myoblasts into myotubes, 90-100% confluent cultures (cultured on standard 6-well plates) were switched to DMEM supplemented with 2% adult horse serum. Myoblast growth and myotube formation were examined using phase-contrast microscopy. The medium was changed every 2 days, and differentiation was allowed to continue for 10 days before the experimentation period.

### Example 2: AT-2 receptor stimulation experiments

L6 myotubes (n=9-12) cultured as described in example 1 were incubated either with angiotensin II (10⁻⁷ mol/l), with a combination of angiotensin II (10⁻⁷ mol/l) and AT-1 receptor blocker losartan (10⁻⁷ mol/L), or with a combination of angiotensin II (10⁻⁷ mol/l), losartan (10⁻⁷ mol/L) and AT-2 receptor blocker PD 123319 (10⁻⁷ mol/L). The uptake of H-3-labeled 2-Deoxy-D-glucose (GE Healthcare Biosciences), which cannot undergo further glycolysis, was measured as follows:
H-3-labeled 2-Deoxy-D-glucose uptake in the differentiated myotubes was analysed as described previously (Ceddia et al., Diabetologia (2005); 48 (1) :132-9) with minor modifications. Differentiated myotubes were serum-starved overnight in DMEM containing 1% glucose before being incubated with angiotensin II, the AT-1 receptor blocker losartan, and/or the AT-2 receptor blocker PD123319. Cells were incubated for 20 minutes with the above-mentioned test chemicals in a reaction solution containing HEPES-buffered (15 mmol/L) Krebs Ringer solution (118 mM NaCl, 4,8 mM KCl, 1,2 mM KH₂PO₄, 0,6 mM MgSO₄ x 7H2O, 0,9 mM CaCl₂ x 2 H₂O) with 0.5% bovine serum albumin. Afterwards 10 µmol/l H-3-labeled 2-deoxy-D-glucose (40 kBq/well) were added to the cells for 10 minutes in the above-mentioned reaction solution also containing the test substances. Subsequently, cells were washed three times with ice-cold phosphate-buffered saline and lysed with 0.1% sodium dodecyl sulphate. Cell lysates were transferred to scintillation vials for radioactivity counting. Non-specific uptake was determined in the presence of cytochalasin B (10 µmol/L) and was subtracted from all values.

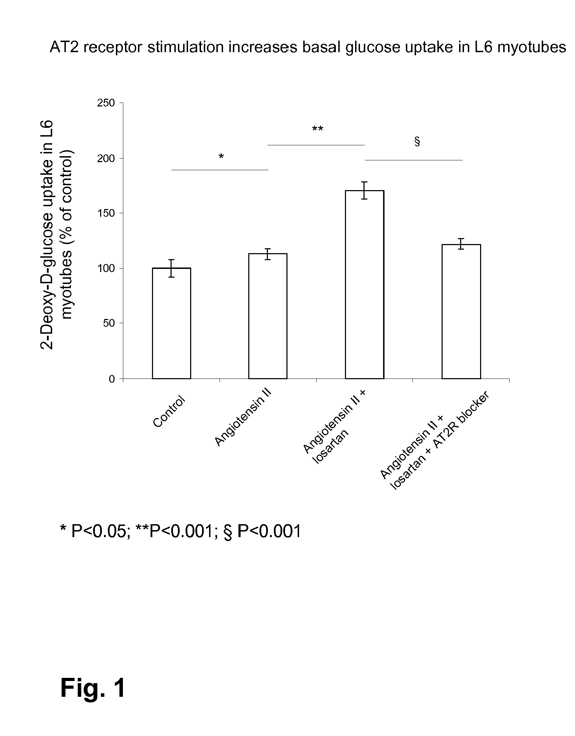

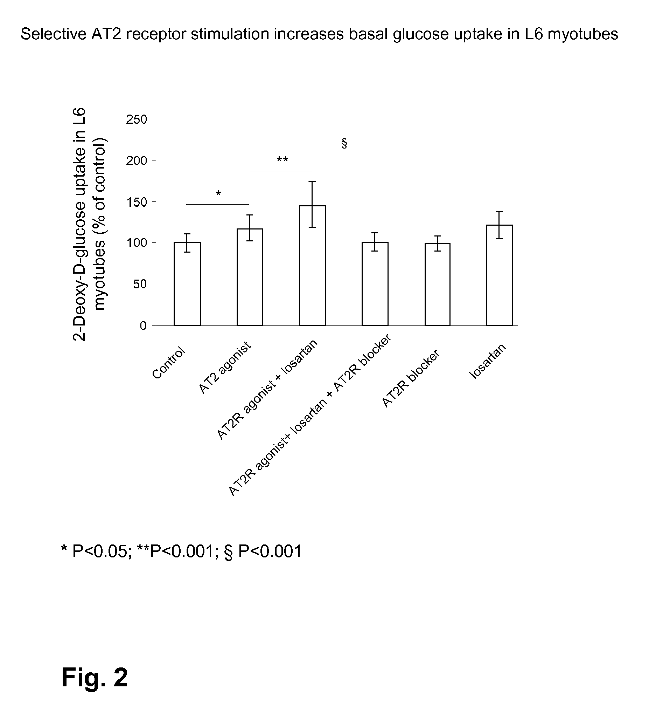

The results are depicted in figure 1. As can be seen in the figure, angiotensin II (10⁻⁷ mol/L) alone induced basal uptake of H-3-labeled 2-Deoxy-D-glucose. However, in the presence of the AT-1 receptor blocker losartan (10⁻⁷ mol/L), angiotensin II strongly enhanced glucose uptake in L6 myotubes even exceeding the magnitude of insulin-mediated glucose uptake in this system (131 ± 6%, insulin data not shown). The effect was inhibited by the AT-2 receptor blocker PD 123319 see figure 2). In contrast, the AT-2 receptor blocker PD123319 (10⁻⁷ mol/L) alone did not affect glucose uptake in these cells.

### Example 3: AT-2 receptor stimulation experiments

In a second group of experiments, L6 myotubes (n=9-12) were incubated with AT-2 receptor agonist CGP-42112A (10⁻⁸ mol/L) alone, with AT-1 receptor blocker losartan (10⁻⁷ mol/L) alone, with AT-2 receptor blocker PD 123319 (10⁻⁷ mol/L) alone, with a combination of AT-2 receptor agonist CGP-42112A (10-8 mol/L) and losartan (10⁻⁷ mol/L), and with a combination of AT-2 receptor agonist CGP-42112A (10⁻⁸ mol/L), losartan (10⁻⁷ mol/L), and PD 123319 (10⁻⁷ mol/L). The uptake of H-3-labeled 2-Deoxy-D-glucose was measured as described in Example 1 above.

The results are depicted in figure 2, showing that the effect of angiotensin II was mimicked by the AT-2 receptor agonist CGP-42112A. This effect was completely blocked by the AT-2 receptor blocker PD123319. Thus, experimental evidence from L6 myotubes refers to a direct effect of pharmacological AT-2 receptor stimulation on glucose uptake in skeletal muscle cells.

### Example 4: Long-term stimulation of the AT-2 receptor

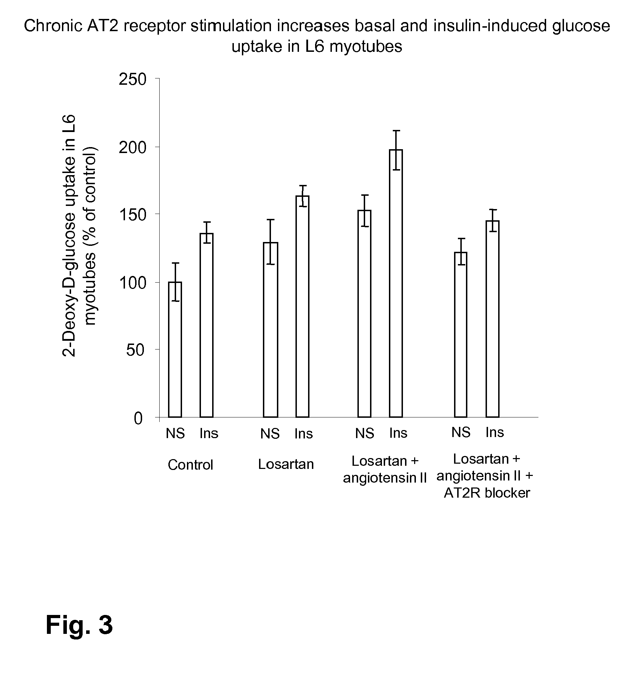

The effect of long-term pharmacological AT-2 receptor stimulation and/or AT-1 receptor blockade on basal and insulin-mediated uptake of H-3-labeled 2-Deoxy-D-glucose in L6 myotubes was tested. For this purpose, L6 myotubes (n=9-12) were incubated for 24 hours with the AT-1 receptor blocker losartan (10⁻⁷ mol/L) alone, with a combination of angiotensin II (10⁻⁷ mol/L) and losartan (10⁻⁷ mol/L), and with a combination of angiotensin II (10⁻⁷ mol/L), AT-2 receptor blocker PD 123319 (10⁻⁷ mol/L) and losartan (10⁻⁷ mol/L). Insulin was added as indicated in figure 3 at a concentration of 1 µg/ml.

The results are depicted in figure 3. The AT-1 receptor blocker losartan induced basal and insulin-mediated glucose uptake of L6 myotubes. However, angiotensin II in the presence of losartan strongly enhanced glucose uptake, an effect which was completely absent in cells which were additionally treated with the AT-2 receptor blocker PD123319. Thus, long-term pharmacological AT-2 receptor stimulation in presence or absence of the AT-1 receptor blocker losartan enhances basal glucose uptake and insulin sensitivity in skeletal muscle cells.

## Claims

1. Use of an agonist of the angiotensin receptor type-2 for the preparation of a pharmaceutical composition for the treatment or prevention of insulin resistance and/or disorders associated with insulin resistance.

2. Use of claim 1, wherein the agonist of the angiotensin receptor type-2 is an antibody or antibody fragment, a small molecule organic compound, a polypeptide or a peptide.

3. Use of claim 2, wherein the agonist of the angiotensin receptor type-2 is CGP-42112A.

4. Use of claim 2, wherein the agonist of the angiotensin receptor type-2 comprises the amino acid sequence of SEQ ID NO:2 or an agonistically active fragment or homolog thereof.

5. Use of claims 1 to 4, wherein the pharmaceutical composition for the treatment of insulin resistance further comprises an antagonist of the angiotensin receptor type-1.

6. Use of claim 5, wherein the antagonist of the angiotensin receptor type-1 is selected from the group consisting of losartan, candesartan, eprosartan, irbesartan, olmesartan, tasosartan, telmisartan, and valsartan.

7. Use of claims 1 to 6, wherein the disorder associated with insulin resistance is selected from diabetes mellitus type 2, diabetic retinopathy, diabetic nephropathy, blindness, cardiovascular abnormalities, hypertension, obesity, dyslipidemia, atherosclerosis, renal failure, neuropathy, syndrome X, acromegaly, and Cushing's syndrome.

8. Pharmaceutical composition comprising an agonist of the angiotensin receptor type-2 for the treatment or prevention of insulin resistance and/or disorders associated with insulin resistance.

9. Pharmaceutical composition of claim 8, wherein the agonist of the angiotensin receptor type-2 is an antibody or antibody fragment, a small molecule organic compound, a polypeptide or a peptide.

10. Pharmaceutical composition of claim 9, wherein the agonist of the angiotensin receptor type-2 is CGP-42112A.

11. Pharmaceutical composition of claim 9, wherein the agonist of the angiotensin receptor type-2 comprises the amino acid sequence of SEQ ID NO:2 or an agonistically active fragment or homolog thereof.

12. Pharmaceutical composition of claims 8 to 11, wherein the pharmaceutical composition for the treatment of insulin resistance further comprises an antagonist of the angiotensin receptor type-1.

13. Pharmaceutical composition of claim 12, wherein the antagonist of the angiotensin receptor type-1 is selected from the group consisting of losartan, candesartan, eprosartan, irbesartan, olmesartan, tasosartan, telmisartan, and valsartan.

14. Pharmaceutical composition of any of claims 8-13, wherein the disorder associated with insulin resistance is selected from diabetes mellitus type 2, diabetic retinopathy, diabetic nephropathy, blindness, cardiovascular abnormalities, hypertension, obesity, dyslipidemia, atherosclerosis, renal failure, neuropathy, syndrome X, acromegaly, and Cushing's syndrome.

15. Pharmaceutical composition of any of claims 8-14, wherein the composition is for administration to a human patient having an age of between 5 and 18 years.


SEQUENCE LISTING

<110>  Universitätsklinikum Hamburg-Eppendorf

<120>  Compositions for treating insulin resistance

<130>  P 80813

<160>  2

<170>  PatentIn version 3.3

<210>  1
<211>  10
<212>  PRT
<213>  Homo sapiens

<400>  1

Asp Arg Val Tyr Ile His Pro Phe His Leu
1               5                   10


<210>  2
<211>  8
<212>  PRT
<213>  Homo sapiens

<400>  2

Asp Arg Val Tyr Ile His Pro Phe
1               5